## Europäisches Patentamt
## European Patent Office
## Office européen des brevets

(19)

(11) Publication number: **0 048 035**
**B1**

(12)

# EUROPEAN PATENT SPECIFICATION

(45) Date of publication of patent specification: **07.12.83**

(51) Int. Cl.³: **C 12 Q 1/40, C 12 P 19/22**

(21) Application number: **81107861.7**

(22) Date of filing: **06.06.79**

(60) Publication number of the earlier application in accordance with Art. 76 EPC: **0005867**

(54) **Maltodextrin phosphorylase limit dextrin and method of producing it.**

(30) Priority: **06.06.78 US 912986**

(43) Date of publication of application:
**24.03.82 Bulletin 82/12**

(45) Publication of the grant of the patent:
**07.12.83 Bulletin 83/49**

(84) Designated Contracting States:
**DE FR GB IT**

(56) References cited:
**DE - A - 2 705 859**
**DE - A - 2 729 636**

(73) Proprietor: **FLOW GENERAL, INC.**
**7655 Old Springhouse Road**
**Mclean Virginia 22102 (US)**

(72) Inventor: **Nix, Paul Thomas**
**62 Forest Drive**
**Jackson N.J. 08627 (US)**
Inventor: **Goldfarb, Rebecca Dickstein**
**2801 North Calvert Street, Apartment No. 1**
**Baltimore, Maryland 21218 (US)**
Inventor: **Stong, Linda Jean**
**6 Blue Spruce Court R.D. 1**
**Hightstown N.J. 08520 (US)**
Inventor: **Sulick, Lorraine Ellen**
**14 Winding Way**
**Short Hills, N.J. 07078 (US)**
Inventor: **Trivedi, Ramesh Chandulal**
**110 W.Main Street**
**Freehold N.J. 07728 (US)**
Inventor: **Morgenstern, Stanley Walter**
**3805, Route 33**
**Neptune N.J. 07753 (US)**

(74) Representative: **Vossius Vossius Tauchner**
**Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 048 035**

## Maltodextrin phosphorylase limit dextrin and method of producing it

This invention relates to maltodextrin phosphorylase (MDP) limit dextrin and to a method for producing it. The MDP limit dextrin is intended for use in reagent compositions to determine alpha-amylase concentrations in body fluids such as plasma, serum, urine and saliva, especially for the detection and measurement of alpha-amylase using coupled enzymatic reactions and the substrate for alpha-amylase that is used therein.

Alpha-amylase determinations are increasingly being used in clinical laboratories as an aid in diagnosing pancreatic and other diseases which alter the normal concentration of alpha-amylase in a body fluid. Alpha-amylase is the common traditional name for the enzyme more precisely designated as alpha-1,4-glucan 4-glucanohydrolase.

Alpha-amylase plays a vital physiological role in enabling the digestion of starch and other polysaccharides by virtue of its specific enzymatic activity in the hydrolysis of alpha-1,4 linkages in the polysaccharide molecule, with the formation of maltose and various other oligosaccharides as ultimate products of the enzymatic reaction. Detection of abnormalities in the alpha-amylase content of body fluids is an important aid in clinical diagnosis.

A number of analytical methods for the determination of alpha-amylase have been developed based upon the ability of alpha-amylase to hydrolyze starch. In all of these methods starch or a starch derivative is digested with the sample containing alpha-amylase to be analyzed. The extent of the alpha-amylase induced degradation can then be measured by a variety of procedures. For example, the decrease in the amount of the starch substrate has been directly measured by an amyloclastic method in which the decreased starch content is determined by adding iodine to the sample and measuring the intensity of the blue-colored starch-iodine complex which is formed. Alternatively, the decrease in starch content has been measured by the decrease in turbidity of an aqueous starch suspension following alpha-amylase digestion. Another chemical method for determining alpha-amylase activity involves the use of a modified starch as the substrate, the starch having a chromophore covalently linked to it. On digestion with alpha-amylase, small water-soluble colored fragments are formed, and the intensity of the color in the solution is measured photometrically.

Yet another chemical analytical procedure is based upon reduction-oxidation analysis for reducing ends which are formed by the reaction of alpha-amylase with the starch substrate.

None of these chemical methods has proven entirely satisfactory for clinical use. All are time-consuming and require standard curves, while most also require the use of blanks and long incubation times. Other problems include variability depending upon the source of the starch, sensitivities due to differences in laboratory technique and the need for specialized equipment.

Still further analyses are based upon enzymatic detection methods and, to date, all prior commercially available enzymatic alpha-amylase kits have been based upon measurements of the rate at which maltose is formed by the action of alpha-amylase on starch or an oligosaccharide substrate such as maltotetraose or maltopentose. In one such analysis maltose is converted to glucose by the enzyme alpha-glucosidase, the level of glucose is in turn determined by conversion to glucose-6-phosphate by hexokinase, and conversion of glucose-6-phosphate to 6-phosphogluconate by reaction with beta-nicotinamide-adenine-dinucleotide (NAD) in the presence of glucose-6-phosphate dehydrogenase, as represented by the following sequence:

$$(1)\ \text{Maltose} \xrightarrow{\text{alpha-glucosidase}} \text{glucose.}$$

$$(2)\ \text{Glucose} + \text{ATP} \xrightarrow{\text{hexokinase}} \text{glucose-6-phosphate} + \text{ATP.}$$

$$(3)\ \text{Glucose-6-phosphate} + \text{NAD} \xrightarrow{\text{glucose-6-phosphate dehydrogenase}} \text{6-phospho-gluconate} + \text{NADH.}$$

In this reaction system, and elsewhere herein and in the claims, the conventional abbreviation NAD is used in reference to the coenzyme beta-nicotinamide-adenine-dinucleotide, and NADH is used in reference to the same coenzyme in its reduced form. It should also be noted that the NAD may be replaced by or used in admixture with NADP, namely beta-nicotinamide-adenine-dinucleotide phosphate, which in its reduced form is indicated by NADPH.

Still other procedures can be used to detect the formation of maltose via the enzyme maltose phosphorylase, as is illustrated by the following sequence:

$$(1)\ \text{Maltose} + \text{phosphate} \xrightarrow{\text{maltose phosphorylase}} \text{glucose} + \text{beta-glucose-1-phosphate.}$$

2

**0 048 035**

$$\text{(2) Beta-glucose-1-phosphate} \xrightarrow{\text{betaphosphoglucomutase}} \text{glucose-6-phosphate.}$$

$$\text{(3) Glucose-6-phosphate+NAD} \xrightarrow[\text{dehydrogenase}]{\text{glucose-6-phosphate}} \text{6-phosphogluconate+NADH.}$$

Typically, the increase in absorbence at 340 nm due to increased levels of NADH is measured photometrically. These methods are still not as accurate or as sensitive as might be desired.

Still more recently, it has been proposed to use as the substrate for the alpha-amylase a starch which has been subjected to partial oxidation, with resulting damaging random effect on alpha-1,4 linked glucoses of the starch molecule to form a so-called "blocked" starch. In such a "blocked" starch the reactivity of the damaged alpha-1,4 linkages is blocked to a sufficient extent to inhibit the activity of an exocarbohydrase on the starch molecule, while at the same time leaving residual alpha-1,4 linkages that are subject to alpha-amylase attack with attendant liberation of free non-reducing chain terminals that can be attacked by an exocarbohydrase such as phosphorylase, which serves as a coupling enzyme in the following enzymatic reaction system:

$$\text{(1) "Blocked" starch} \xrightarrow{\text{alpha-amylase}} \text{"unblocked" starch.}$$

$$\text{(2) "Unblocked" starch+inorganic phosphate} \xrightarrow{\text{phosphorylase}} \text{glucose-1-phosphate +degraded starch.}$$

$$\text{(3) Glucose1-phosphate} \xrightarrow{\text{phosphoglucomutase}} \text{glucose-6-phosphate.}$$

$$\text{(4) Glucose-6-phosphate+NAD} \xrightarrow[\text{dehydrogenase}]{\text{glucose-6-phosphate}} \text{NADH+6-phosphogluconate.}$$

The use of "blocked" starch in providing a substrate comprised in a coupled enzymatic reaction system involves inherent disadvantages in that in producing the blocked starch substrate the oxidation of the starch is only partial and damages alpha-1,4 linkages randomly throughout the starch molecule. As a result, difficulties are encountered as regards controlling the uniformity of the substrate and its reactivity with alpha-amylase and, as a corollary, correctly indicating the rate of change in absorbence occasioned by alpha-amylase in a body fluid. Moreover, because of the reduced overall activity occasioned by the partial oxidation, the amount of the "blocked" starch required for accomplishing a given change in absorbence is undesirably large and substantially interferes with the sensitivity of the test determination (DE—A—2729636 and C. Y. Huang, Clinical Chemistry, 22, 1164 (Article 023) (1976).

It is an object of this invention to provide a method for producing MDP limit dextrin to be used for determining the concentration of alpha-amylase in a body fluid.

Starch consists in nature of a combination of two polymers, alpha-amylose and amylopectin. Alpha-amylose consists essentially of long unbranched chains in which all of the D-glucose units are bound by alpha-1,4 linkages, namely linkages which contain an oxygen atom and which link the first carbon atom of a glucose unit with the fourth carbon atom of an immediately adjacent glucose unit. The chains are polydisperse and vary in molecular weight from a few thousand to around 50,000, there being a free, namely unlinked, non-reducing terminal at one end and a free reducing terminal at the other end.

Amylopectin, unlike amylose, is highly branched. In a typical amylopectin the branches or chains contain on average twelve glucose units successively connected by alpha-1,4 linkages, so that branch points occur on average every twelfth glucose unit. At the branch points the chains of alpha-1,4 linked glucose units are linked respectively to the first, fourth and sixth atoms of a single glucose unit, the occurrence of the 1—6 linkage with the sixth carbon atom being the characterizing structural feature at the branch points. The molecular structure of amylopectin normally comprises at its periphery a multiplicity of branches that are linked at one end to branch points and have a free non-reducing terminal at the remote end with the exception of a single branch that has a free reducing terminal at the remote end. Amylopectin is further characterized by a polysaccharide core comprised of a multiplicity of chains and branches made up of alpha-1,4 linked glucose units, which chains interconnect and are terminally linked at each end to branch points and therefore do not have a free non-reducing terminal or a free reducing terminal.

When amylose is hydrolyzed by the action of alpha-amylase the resulting cleavage of the alpha-1,4 linkages may occur anywhere along the amylose chain in such a way as to ultimately yield a

3

mixture of oligosaccharides and maltose. Because of its ability to cleave interior linkages as well as linkages occurring near free non-reducing terminals alpha-amylase is referred to as an endocarbohydrase. Amylose may also be attacked by enzymes known as exocarbohydrases, such as beta-amylase, glucoamylase and various phosphorylases. These enzymes cleave away successive carbohydrate monomers beginning only at a free non-reducing terminal of a chain of successive alpha-1,4 linked glucose units.

Because of its branched molecular structure, amylopectin is attacked in different ways by alpha-amylase and by exocarbohydrases. Exocarbohydrases cannot hydrolyze the alpha-1,6 linkages that occur at the branch points. Alpha-amylase, being an endocarbohydrase, has the ability to cleave alpha-1,4 linkages not only near free non-reducing chain terminals but also randomly at alpha-1,4 linkages between the chain terminals in the interior core portion of amylopectin. Exocarbohydrases, on the other hand, can only attack alpha-1,4 linked glucopolysaccharides that contain free non-reducing terminals so as to cleave off a carbohydrate monomer followed by successively cleaving off additional monomers while progressing towards the alpha-1,6 branch point of an attacked branch. Eventually the enzymatic activity of an exocarbohydrase on starch will cease.

The molecularly large carbohydrate product that results from the exhaustive reaction of amylopectin with an exocarbohydrate is known in the art as a limit dextrin (Biochem., *76*, 264 (1960)). In general the structure of the limit dextrin may be considered as an amylopectin core comprising alpha-1,6 branch sites interconnected by chains of alpha-1,4 glucose that are terminally linked to the alpha-1,6 branch sites. However, the ability of various exocarbohydrases to attack the chains having free non-reducing terminals varies; as a consequence, depending on the exocarbohydrase employed, the amylopectin core will have attached to it short residual alpha-1,4 glucose chains of different lengths. In other words, the limit dextrin obtained with one exocarbohydrase, e.g. beta-amylase, is not identical to a limit dextrin obtained with another exocarbohydrase, e.g. maltodextrin phosphorylase. Beta-amylase, for example, is less effective than maltodextrin phosphorylase in attacking the alpha-1,4 linked glucose chains. Accordingly, the limit dextrin obtained with beta-amylase has longer residual chains than the limit dextrin obtained with maltodextrin phosphorylase. One consequence of this difference in enzyme activity is that the limit dextrin obtained with beta-amylase, although no longer capable of acting as a substrate for beta-amylase, is capable of acting as a substrate for maltodextrin phosphorylase. On the other hand, the limit dextrin obtained with maltodextrin phosphorylase cannot act as a substrate for either beta-amylase or maltodextrin phosphorylase. The limit dextrin of this invention is one which will not serve as a substrate for maltodextrin phosphorylase.

The MDP limit dextrin of this invention need not necessarily be prepared from starch; it may, for example, be produced from glycogen, which is a storage polysaccharide found in animal tissues and is especially abundant in liver and muscle tissue. Glycogen is similar to amylopectin in that it is a polysaccharide characterized by chains of alpha-1,4 linked glucose units. However, it is more highly branched, with alpha-1,6 branch points occurring at intervals of about every 8 to 10 glucose units. As in the case of amylopectin, glycogen contains chains of alpha-1,4 linked glucose units, the chains having their terminal extremities linked to the branch points. Accordingly, when glycogen is exhaustively treated with an exocarbohydrase the resulting product is a limit dextrin essentially similar to that produced from starch with that exocarbohydrase.

MDP limit dextrin is employed as the substrate for alpha-amylase comprised in a specimen to be analyzed, such as a sample of serum or urine, thereby initiating a series of reactions resulting in a chromophore response, the amount of the alpha-amylase being rate limiting and the rate of the chromophore response being a measure of the concentration of the alpha-amylase in the specimen.

When employing MDP Limit Dextrin as the substrate for an alpha-amylase assay, attack of the alpha-amylase on the substrate occurs in the presence of the maltodextrin phosphorylase reagent of the assay system. It is inherent from the molecular structure of MDP Limit Dextrin that the attack on it by alpha-amylase in the specimen is limited essentially to ruptures of the chains of alpha-1,4 linked glucose unit that have their terminal extremities linked to the alpha-1,6 branch points therein, with formation of polysaccharide or oligosaccharide fragments of MDP Limit Dextrin having newly formed free non-reducing chain terminals which are available for reaction with the maltodextrin phosphorylase. The maltodextrin phosphorylase is provided in excess and in sufficient amounts to keep the alpha-amylase in the specimen being analyzed as the rate limiting constituent, whereby, as each new free non-reducing terminal is formed from the MDP Limit Dextrin substrate, it is attacked by the maltodextrin phosphorylase. The concentrations of reagents in the analysis system are adjusted so that the rate of the reaction of alpha-amylase with MDP, Limit Dextrin is the one being measured.

As is well-known, maltodextrin phosphorylase can be derived from mutant *E. coli*. See, for instance, Buehner, Crystallization and Crystallographic Data of *Escherichia Coli* Maltodextrin Phosphorylase, FEBS Letters, Vol. 85, No. 1, 914 (1978) and Thanner et al., Hydrophobic and Biospecific Chromatography in the Purification of Maltodextrin Phosphorylase from *E. Coli*, FEBS Letters, Vol. 55, No. 1, 178 (1975). However, maltodextrin phosphorylase obtained from other sources may be used in the practice of this invention.

The MDP limit dextrin is prepared by a relatively simple and inexpensive procedure which provides a substrate that meets specifications with a high degree of uniformity. Basically the preparation of the

0 048 035

limit dextrin comprises incubating the selected carbohydrate with maltodextrin phosphorylase in an aqueous medium in the presence of inorganic phosphate until the action of the phosphorylase has become exhaustive, and then heating the aqueous medium to boiling to destroy the phosphorylase. Preferably the initial incubation is performed when the aqueous medium is at a temperature of about 37°C. Since the reaction is enzymatically induced, the concentration of the phosphorylase is not critical and may be varied over a wide range such as 1 to 20,000 U when used with a solution containing 50 g/liter of starch. However, it is essential to remove the glucose-1-phosphate formed in the production of the MDP limit dextrin before it can be used in the assay reaction system. This may be accomplished by dialysis.

Ordinarily the carbohydrate from which the limit dextrin is produced is starch in the form referred to in the art as soluble starch, that is starch that has been heated with acid followed by removal of most of the amylose.

As has been noted above, the limit dextrin formed by exhaustive reaction upon soluble starch or other carbohydrate source using beta-amylase is not identical with that formed using maltodextrin phosphorylase in the presence of inorganic phosphate since, as between these exocarbohydrases, the final extent of the cleavage starting with non-reducing chain terminals is not identical. It is an additional feature of this invention that the MDP limit dextrin used in the assay is preferably produced in a two-step process wherein the starch is initially incubated with beta-amylase followed by incubation with maltodextrin phosphorylase in the presence of inorganic phosphate. It has been found that when the limit dextrin is produced in this way the accuracy of the assay is substantially improved. This is because most of the cleavage of the carbohydrate source is effected by beta-amylase, and maltose is the major "by-product" of this reaction. Since maltose does not affect the analysis, the presence of maltose as an impurity is not a particular problem. On the other hand, the glucose-1-phosphate "by-product" formed by incubation of the carbohydrate source with maltodextrin phosphorylase can substantially interfere with the analysis. Thus the use of maltodextrin phosphorylase only as the final step in the production of MDP limit dextrin reduces the amount of glucose-1-phosphate which is formed and minimizes the amount of purification of the MDP limit dextrin which is required before use in accordance with this invention.

The MDP limit dextrin as initially produced in an aqueous medium may be employed by adding it to the aqueous medium comprising the remaining components of the overall reaction system of the assay. For convenience of handling the MDP limit dextrin may be reduced to the solid state by lyophilizing. It may be lyophilized *per se* or in combination with one or more components of the reaction system that is employed. When employed in combination, the basic combination used in any of the reaction systems is the MDP limit dextrin with the maltodextrin phosphorylase. To the basic combination may be added any one or more of the remaining components of the selected coupled enzymatic reaction system. All the remaining reaction components are preferably present in the lyophilized state so that at the time the assay is performed all that is required is the addition of water to form the aqueous medium of desired concentration and pH to which the alpha-amylase specimen may be added when the aqueous medium is at the desired temperature. However, if desired, the various components may be provided either individually or in subcombinations as a kit for admixture at the time the assay is carried out, so that the coupled reactions involved may occur simultaneously to form the chromophore at a rate which is limited by the alpha-amylase added to complete the reagent system and thereby to trigger the reactions involved.

The following example is illustrative of the preferred practice of the invention. The following combination is initially prepared:

| | |
|---|---|
| Starch solution | 50 g/liter |
| Phosphate buffer | 50 mM |
| Beta-amylase | 7200 U. |
| pH | 6.9 |

The foregoing composition is incubated for 18 hours at 37°C. 2100 U. of maltodextrin phosphorylase are then added and incubation is continued at 37°C for 24 hours. Thereafter, the mixture is heated to boiling to destroy the beta-amylase and the phosphorylase, and then dialyzed to remove glucose-1-phosphate.

**Claims**

1. A method of producing maltodextrin phosphorylase (MDP) limit dextrin suitable for forming a reagent composition to be used in an alpha-amylase assay, comprising exhaustively treating a carbohydrate selected from starch, glycogen or mixtures thereof in an aqueous medium with beta-amylase, followed by further treating the so-treated carbohydrate with maltodextrin phosphorylase in the presence of inorganic phosphate and destroying said beta-amylase and said phosphorylase after said treatment therewith by heating said aqueous medium.

2. The method according to claim 1, further comprising removing any glucose-1-phosphate

5

formed during the treatment with maltodextrin phosphorylase, thereby separating it from the limit dextrin.

3. A method according to claim 2 wherein said glucose-1-phosphate is removed by dialysis against a phosphate buffer solution.

4. MDP limit dextrin which is the product of the method of claim 2.

**Patentansprüche**

1. Verfahren zur Herstellung von Maltodextrin-Phosphorylase (MDP) Grenzdextrin, das geeignet für ein Reagenz zur Bestimmung von alpha-Amylase ist, dadurch gekennzeichnet, daß man Stärke oder Glycogen oder ein Gemisch davon in einem wäßrigen Medium mit Beta-Amylase erschöpfend behandelt, darauf mit Maltodextrin-Phosphorylase in Gegenwart von anorganischem Phosphat weiterbehandelt und die Beta-Amylase und Phosphorylase nach dieser Behandlung durch Erhitzen des wäßrigen Mediums zerstört.

2. Verfahren nach Anspruch 1, dadurch gekennzeichnet, daß außerdem jegliches während der Behandlung mit Maltodextrin-Phosphorylase gebildete Glucose-1-phosphat entfernt und somit vom Grenzdextrin abgetrennt wird.

3. Verfahren nach Anspruch 2, dadurch gekennzeichnet, daß das Glucose-1-phosphat durch Dialyse gegen eine Phosphat-pufferlösung entfernt wird.

4. MDP Grenzdextrin, dadurch gekennzeichnet, daß es das Verfahrensprodukt von Anspruch 2 ist.

**Revendications**

1. Procédé de production de dextrine limite de phosphorylase de maltodextrine (MDP) approprié à la formation d'une composition de réactifs destinée à être utilisée dans un test d'alpha-amylase comprenant le traitement complet d'un carbohydrate choisi parmi l'amidon, le glycogène ou les mélanges de ceux-ci en milieu aqueux avec la bêta-amylase, suivi d'un traitement ultérieur du carbohydrate ainsi traité avec la phosphorylase de maltodextrine en présence de phosphate minéral et la destruction de ladite bêta-amylase et de ladite phosphorylase après ledit traitement en chauffant ledit milieu aqueux.

2. Procédé selon la revendication 1, caractérisé en ce qu'il comprend en outre l'élimination du glucose-1-phosphate formé pendant le traitement avec le phosphorylase de maltodextrine, ce par quoi on sépare la dextrine limite.

3. Procédé selon la revendication 2, caractérisé en ce que le glucose-1-phosphate est éliminé par dialyse par une solution de tampon phosphate.

4. Dextrine limite de phosphorylase de maltodextrine (MDP) obtenue selon le procédé de la revendication 2.